# EUROPÄISCHE PATENTSCHRIFT

(11) **EP 2 288 305 B1**
(45) Veröffentlichungstag und Bekanntmachung des Hinweises auf die Patenterteilung: **11.07.2012**
(21) Anmeldenummer: 09761363.2
(22) Anmeldetag: 13.05.2009
(51) Int. Cl.: A61B 18/02

(54) **KRYOCHIRURGISCHES GERÄT ZUM BETREIBEN VON KRYOSONDEN, VERFAHREN ZUM BETREIBEN EINER KRYOSONDE**
CRYOSURGICAL DEVICE FOR OPERATING CRYOPROBES, METHOD FOR OPERATING A CRYOPROBE
APPAREIL CRYOCHIRURGICAL POUR FAIRE FONCTIONNER DES CRYOSONDES, ET PROCÉDÉ POUR ACTIONNER UNE CRYOSONDE

(30) Priorität: 12.06.2008 DE 102008028046; 19.08.2008 DE 102008038310
(43) Veröffentlichungstag der Anmeldung: 02.03.2011
(73) Patentinhaber: ERBE Elektromedizin GmbH, 72072 Tübingen (DE)
(72) Erfinder: GEISELHART, Franz, 72770 Reutlingen (DE); GROSS, Stefan, 72072 Tübingen (DE)
(74) Vertreter: Bohnenberger, Johannes
(86) Internationale Anmeldenummer: PCT/EP2009/003407
(87) Internationale Veröffentlichungsnummer: WO 2009/149800

(56) Entgegenhaltungen:
- WO-A2-2005/038357
- WO-A2-2007/129310
- DE-A1-102006 003 571
- US-A- 625 105
- US-A- 5 674 218
- US-A1- 2004 215 295
- US-A1- 2006 004 350
- US-A1- 2007 156 125

## Beschreibung

Die Erfindung betrifft ein kryochirurgisches Gerät zum Betreiben einer ersten Kryosonde mit einem druckfesten Rücklauf und einer zweiten Kryosonde mit einem drucklosen Rücklauf sowie ein entsprechendes Verfahren.

Die Kryotherapie, umfassend die Kryochirurgie, hat eine Vielzahl von Anwendungen. Kryosonden werden beispielsweise verwendet, um krankhaftes Gewebe zu zerstören, Gewebeproben zu entnehmen und/oder Fremdkörper zu entfernen.

In der Kryotherapie, insbesondere in der Kryochirurgie, wird häufig Kälte mittels einer Sonde appliziert, um somit einen heilenden Effekt zu erzielen.

Um das entsprechende Instrument abzukühlen, gibt es verschiedene Verfahren. Häufig wird der Joule-Thomson-Effekt ausgenutzt. Dabei wird ein Fluid, insbesondere ein Gas - nahe dem Applikationsort über eine Düse expandiert, wodurch das Gas eine Temperaturänderung erfährt. Die Kühlleistung basiert hier unter anderem auf.der Druckdifferenz, die am Ort der Expansion vorliegt. Die Expansion findet in einer Expansionskammer statt. Um eine effektive Kühlung des Instruments zu gewährleisten, ist es notwendig, das expandierte Gas aus der Expansionskammer abzuleiten, ohne einen ungewollten Rückstau zu verursachen.

In der Praxis unterscheidet man zwischen zwei Arten von Sonden, nämlich starren abtaubaren Sonden und flexiblen Sonden. Starre Sonden verfügen über einen starren, druckbeständigen Zulauf und Rücklauf. Eine vorteilhafte Regelung der Kühlleistung lässt sich bei hohem und konstantem Zulaufdruck über das Einstellen des Rücklaufdrucks gewährleisten. Ein Vorteil von starren Sonden besteht darin, dass sie sich auch zur Erwärmung des Applikationsortes einsetzen lassen.

Flexible Sonden weisen einen druckfesten Zulauf und einen nicht druckfesten Rücklauf auf. Das expandierte Gas wird mitunter im Inneren des Sondenschafts zurückgeleitet. Derartige Sonden sind häufig wesentlich flexibler als eine entsprechende starre Sonde. Sie lassen sich vorteilhaft mit flexiblen Endoskopen bzw. Flexoskopen verwenden. Jedoch sind die Rückleitungen oder Rückführungen des Fluids derart beschaffen, dass diese lediglich einen geringen Staudruck verkraften. Daher wird die Kühlleistung von flexiblen Sonden über den Zulaufdruck geregelt. Nach der Expansion des Fluids in der Expansionskammer kann das Fluid mit geringem Strömungswiderstand abfließen (druckloser Rücklauf).

Es ist wünschenswert, beide Arten von Sonden an einem Gerät zu betreiben. Ein entsprechendes kryochirurgisches Gerät umfasst üblicherweise eine Fluidquelle, beispielsweise eine Gasflasche mit einem Arbeitsgas (z.B. CO₂ oder N₂O). Eine Druckeinstelleinrichtung zum Einstellen eines geeigneten Drucks an dem Zulauf und/oder Rücklauf und eine Steuerung, die die Druckeinstellvorrichtung derart steuert, dass eine konstante und/oder reproduzierbare Kühlleistung bereitgestellt wird.

Aus der DE 10 2006 003 571 A1 ist ein entsprechendes kryochirurgisches Gerät bekannt, das sowohl starre als auch flexible Sonden betreiben kann. Die Regelung der Kühlleistung der angeschlossenen Sonden durch das Gerät ist unzureichend. Es ist nicht möglich, auf unterschiedliche Versorgungsdrücke und temperaturbedingte Änderungen zu reagieren.

Die US 2007/0156125 A1 beschreibt eine Kryobiopsiesonde.

Ausgehend von diesem Stand der Technik, insbesondere der DE 10 2006 003 571 A1, ist es Aufgabe der vorliegenden Erfindung, ein kryochirurgisches Gerät zur effizienten Steuerung von Kryosonden bereitzustellen, das einfach ausgebildet ist, gleichzeitig jedoch hohe Sicherheitsstandards erfüllt. Des Weiteren soll ein entsprechendes Verfahren angegeben werden.

Die Aufgabe wird erfindungsgemäß durch das kryochirurgische Gerät gemäß Anspruch 1 sowie durch das Verfahren gemäß Anspruch 14 gelöst.

Insbesondere wird die Aufgabe durch ein kryochirurgisches Gerät zum Betreiben einer ersten Kryosonde mit einem druckfesten Rücklauf und einer zweiten Kryosonde mit einem drucklosen bzw. nicht druckfesten Rücklauf gelöst, wobei das Gerät einen ersten Modus, nämlich einen Hinterdruckregelungsmodus zum Betreiben der ersten Kryosonde und einen zweiten Modus, nämlich einen Vorderdruckregelungsmodus zum Betreiben der zweiten Kryosonde hat, wobei das Gerät umfasst:
- eine Steuerung,
- eine Fluidquelle zum Bereitstellen eines Fluids, insbesondere eines Gases, das zur Kühlung der Kryosonden, insbesondere mittels des Joule-Thomson-Effekts in diese einleitbar ist,
- mindestens zwei Kryosondenanschlüsse, wobei an einen Kryosondenanschluss der Kryosondenanschlüsse im ersten Modus die erste Kryosonde und im zweiten Modus die zweite Kryosonde angeschlossen ist, und
- eine Druckeinstellvorrichtung mit mindestens einem Druckregelventil, die derart mit den Kryosonden verbunden ist, dass durch diese im ersten und zweiten Modus das Druckverhältnis des Zulaufs zum Rücklauf der Kryosonden und/oder der Druck im Zulauf regelbar ist, wobei die Steuerung durch ein Einstellen des Druckregelventils die Kühlleistung der Kryosonden steuert.

Ein zentraler Gedanke der vorliegenden Erfindung besteht also darin, eine möglichst einfache Fluidschaltung bereitzustellen, mit der sich flexible und starre Kryosonden bzw. Kryosonden mit druckfesten oder nicht druckfesten Rückläufen, betreiben lassen. Des Weiteren wird erfindungsgemäß mindestens ein Kryosondenanschluss für beide Sondentypen verwendet. Der Aufwand beim Zuordnen der einzelnen Kryosondenstecker zu den entsprechenden Kryosondenanschlüssen kann somit reduziert werden. Erfindungsgemäß kann eine einzige Druckeinstellvorrichtung mit einem Druckregelventil dazu verwendet werden, um sowohl den Fluiddruck in dem Rücklauf der ersten Kryosonde, als auch den in dem Zulauf der zweiten Kryosonde zu regeln. Somit kann der Aufbau des kryochirurgischen Geräts vereinfacht werden. Das wirkt sich nicht nur auf die Kosten sondern auch auf die Sicherheit des Geräts aus. Es sei noch angemerkt, dass der Begriff "drucklos" derart aufzufassen ist, dass hier ein annähernd atmosphärischer Druck vorliegt. Das Druckregelventil kann ein Proportionalventil oder ein Nadelventil sein.

Bei entsprechender Einstellung des Druckverhältnisses des Zulaufs zum Rücklauf kann bei der ersten Kryosonde ein Abtauen der Sonde gewährleistet werden.

Die Druckeinstellvorrichtung kann im ersten Modus derart mit der ersten Kryosonde verbunden sein, dass durch das Einstellen des Druckregelventils der Druck in dem Zulauf und in dem Rücklauf der ersten Kryosonde regelbar ist. Die bereits genannte vorteilhafte Regelung der Kryosondenleistung entlang der Siede-/Taulinie des Fluids kann auf diese Weise gewährleistet werden. Als weitere Vorteile ergeben sich eine kleine Strömungsgeschwindigkeit, ein geringer Strömungswiderstand, ein geringer Druckverlust an Zu- und Rücklauf und bessere Wärmetauscheffekte am Sondenkopf.

Die Druckeinstellvorrichtung kann im zweiten Modus derart mit der zweiten Kryosonde verbunden sein, dass bei im Wesentlichen konstantem - meist geringem - Druck in dem Rücklauf der zweiten Kryosonde der Druck in dem Zulauf einstellbar ist. Es lässt sich also auch die Kühlleistung von flexiblen Sonden vorteilhaft einstellen.

Die Druckeinstellvorrichtung kann ein 3/2-Proportionalventil umfassen, das mit einem ersten Proportionalventilanschluss bzw. Druckregelventilanschluss an die Fluidquelle, mit einem zweiten Proportionalventilanschluss bzw. Druckregelanschluss an die Entlüftungseinrichtung und mit einem dritten Proportionalventilanschluss bzw. Druckregelventilanschluss an mindestens einen der Kryosondenanschlüsse angeschlossen ist. Vorzugsweise handelt es sich bei diesem mindestens einen Kryosondenanschluss um den Anschluss, der sowohl von der ersten Kryosonde als auch von der zweiten Kryosonde genutzt wird. Durch das Verwenden eines 3/2-Proportionalventils, also eines Ventils mit drei Anschlüssen und zwei Hauptstellpositionen, lässt sich die Druckeinstellvorrichtung sehr einfach aufbauen. Das 3/2-Proportionalventil dient zur Einstellung des Drucks im Rücklauf der ersten Kryosonde und des zugeführten Gasvolumens in den Zulauf der zweiten Kryosonde.

Das kryochirurgische Gerät kann eine Schaltvorrichtung mit mindestens einem Schaltventil umfassen, die für einen Wechsel zwischen dem ersten und dem zweiten Modus mit der Druckeinstellvorrichtung und mindestens einem Kryosondenanschluss verbunden ist.

Vorzugsweise ist auch dieser Kryosondenanschluss der Kryosondenanschluss, der sowohl mit der ersten Sonde als auch mit der zweiten Sonde verbunden ist. Das Schaltventil stellt eine derartige Verbindung zwischen dem Kryosondenanschluss und der Druckeinstellvorrichtung her, dass im ersten Modus der Druck in dem Rücklauf, und im zweiten Modus der Druck im Zulauf durch die Druckeinstelleinrichtung, insbesondere durch das dortige Druckregelventil einstellbar ist.

Die Steuerung kann die Schaltvorrichtung aktivieren. Somit kann ein automatisches Wechseln zwischen den Steuerzuständen, also dem ersten Modus und dem zweiten Modus, gewährleistet werden. Der Arzt oder die bedienende Person müssen beim Anschluss einer ersten oder zweiten Kryosonde nicht darauf achten, den entsprechenden Moduls einzustellen.

Dies ist besonders vorteilhaft, wenn die Steckverbindungen zu den Sonden derart ausgestaltet sind, dass eine entsprechende Zuordnung entsprechend dem Sondentyp erfolgt.

Es ist möglich, die Schaltvorrichtung durch mehrere, insbesondere zwei 2/2-Schaltventile (ein Ventil mit zwei Anschlüssen und zwei Stellpositionen) herzustellen. Vorteilhafter ist es aber, wenn die Schaltvorrichtung ein 3/2-Schaltventil (drei Anschlüsse, zwei Stellpositionen) umfasst, das mit einem ersten Schaltventilanschluss an die Druckeinstellvorrichtung, mit einem zweiten Schaltventilanschluss an die Entlüftungseinrichtung und mit einem dritten Schaltventilanschluss an einen Kryosondenanschluss angeschlossen ist. Der Aufbau der Vorrichtung kann somit weiter vereinfacht werden.

Das kryochirurgische Gerät kann mindestens einen Drucksensor zum Bestimmen eines Eingangsdrucks und/oder eines Ausgangsdrucks der Druckeinstelleinrichtung umfassen. Somit ist ein automatisches Regeln der Kühlleistung durch die Steuerung möglich.

Vorzugsweise ist das Fluid aus Kohlendioxid und/oder Stickoxidul (Lachgas) oder ein Gemisch dieser Gase. Diese Gase weisen einen hohen Joule-Thomson-Koeffizienten auf und sind bei Normaltemperatur verflüssigbar. Es kann aber auch jedes andere Gas verwendet werden, dessen Joule-Thomson-Inversionstemperatur oberhalb der Körpertemperatur des Patienten liegt.

Das kryochirurgische Gerät kann eine Detektionseinrichtung umfassen, die feststellt, ob eine erste Kryosonde oder eine zweite Kryosonde an die Kryosondenanschlüsse angeschlossen sind. Die Detektionseinrichtung ist mit der Steuerung verbunden und sendet Gerätekennzeichensignale, die es der Steuerung ermöglichen festzustellen, ob eine Kryosonde vom ersten Typ (erste Kryosonde) oder vom zweiten Typ (zweite Kryosonde) angeschlossen ist. Hierdurch erhöht sich der Bedienerkomfort des Geräts weiter. Die Steuerung kann so bestimmen, welche Kryosondenanschlüsse belegt sind und den entsprechenden Modus einstellen. Die Detektionseinrichtung kann auch als Sicherungseinrichtung dienen, die das Vorhandensein eines pneumatischen Kontakts zwischen mindestens einem Kryosondenanschluss und einer Kryosonde detektiert. Wird die pneumatische Verbindung unterbrochen, so unterbricht die Sicherungseinrichtung die Fluidzufuhr. Auf diese Weise kann das Austreten von Fluid in die Umwelt effizient vermieden werden.

Das kryochirurgische Gerät kann einen Flowsensor zur Bestimmung der Kühlleistung der angeschlossenen Kryosonde aufweisen. Durch die Bestimmung der Fluidmenge, die die Kryosonde durchströmt, lassen sich Rückschlüsse über die in der Kryosonde aufgebrachte Kühlleistung ziehen. Auch ist es möglich, die Temperatur durch ein Regeln und Messen der Fluidmenge einzustellen bzw. zu bestimmen.

Des Weiteren wird die besagte Aufgabe durch ein Verfahren zum Betreiben einer Kryosonde mit einem kryochirurgischen Gerät gelöst, wobei das Gerät ein Fluid zum Kühlen der Kryosonde bereitstellt, umfassend die Schritte:
a) Bestimmen, ob eine erste Kryosonde mit einem starren oder druckfesten Rücklauf oder eine zweite Kryosonde mit einem flexiblen oder drucklosen Rücklauf angeschlossen ist;
b) Einstellen eines ersten Modus (Gegendruckmodus oder Hinterdruckregelungsmodus), wenn die erste Kryosonde angeschlossen ist, und Einstellen eines zweiten Modus (Freilaufmodus oder Vorderdruckregelungsmodus), wenn die zweite Kryosonde angeschlossen ist;
c) Steuern einer Druckeinstellvorrichtung mit einem Druckregelventil zum Regeln eines Druckverhältnisses zwischen Zulauf und Rücklauf der Kryosonden; wobei der Schritt b) eine derartiges Einstellen einer Schaltvorrichtung umfasst, dass im ersten Modus zumindest der Rücklauf der ersten Kryosonde mit einem Druckregelventilanschluss des Proportionalventils verbunden ist und im zweiten Modus zumindest der Zulauf der zweiten Kryosonde mit einem Druckregelventilanschluss des Proportionalventils verbunden ist.

Auch hier wird also mittels eines Proportionalventils das Druckverhältnis im Zulauf und Rücklauf der ersten Kryosonde oder der zweiten Kryosonde vorteilhaft geregelt.

Weitere vorteilhafte Ausführungsformen ergeben sich aus den Unteransprüchen.

Nachfolgend wird die Erfindung anhand von einigen Ausführungsbeispielen beschrieben, die mittels Abbildungen näher erläutert werden. Hierbei zeigen:
- Fig. 1 die Komponenten eines kryochirurgischen Geräts gemäß der Erfindung;
- Fig. 2 einen ersten Fluidschaltplan für das kryochirurgische Gerät;
- Fig. 3 einen Fluidflussplan des Fluidschaltplans aus Fig. 2 bei maximaler Kühlleistung für eine flexible Kryosonde;
- Fig. 4 einen Fluidflussplan des Fluidschaltplans aus Fig. 2 bei maximaler Kühlleistung für eine starre Kryosonde;
- Fig. 5 einen zweiten Fluidschaltplan für das kryochirurgische Gerät;
- Fig. 6 einen Fluidflussplan des Fluidschaltplans aus Fig. 5 bei maximaler Kühlleistung für eine flexible Kryosonde;
- Fig. 7 einen Fluidflussplan des Fluidschaltplans aus Fig. 5 bei maximaler Kühlleistung für eine starre Kryosonde;
- Fig. 8 einen dritten Fluidschaltplan für das kryochirurgische Gerät;
- Fig. 9 einen Fluidflussplan des Fluidschaltplans aus Fig. 8 bei maximaler Kühlleistung für eine flexible Kryosonde;
- Fig. 10 einen Fluidflussplan des Fluidschaltplans aus Fig. 8 bei maximaler Kühlleistung für eine starre Kryosonde;
- Fig. 11 einen vierten Fluidschaltplan für das kryochirurgische Gerät;
- Fig. 12 einen Fluidflussplan des Fluidschaltplans aus Fig. 11 bei maximaler Kühlleistung für eine flexible Kryosonde; und
- Fig. 13 einen Fluidflussplan des Fluidschaltplans aus Fig. 11 bei maximaler Kühlleistung für eine starre Kryosonde.

In der nachfolgenden Beschreibung werden für gleiche und gleich wirkende Teile dieselben Bezugsziffern verwendet.

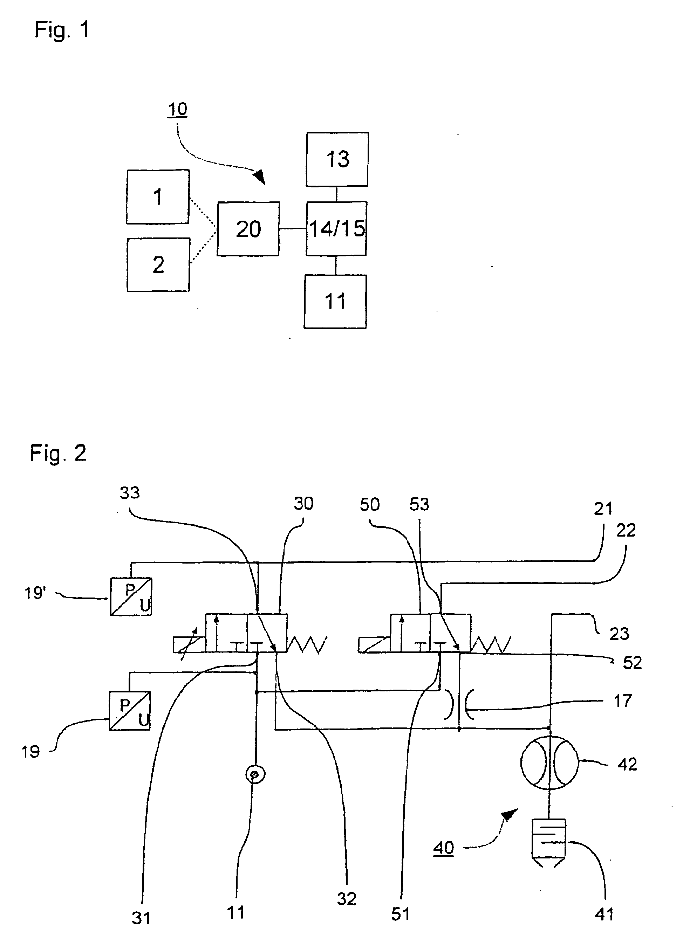

Ein erfindungsgemäßes kryochirurgisches Gerät 10 setzt sich, wie in Fig. 1 gezeigt, aus einer Fluidquelle 11 (z.B. einer Gasflasche) zum Bereitstellen von Fluid mit konstantem Druck, einer Schaltvorrichtung 15 und einer Druckeinstellvorrichtung bzw. -einrichtung 14 zusammen. Des Weiteren ist eine Steuerung 13 vorgesehen, die die Druckeinstellvorrichtung 14 und die Schaltvorrichtung 15 steuert, um angeschlossene Kryosonden 1, 2 mit dem nötigen Fluid zu betreiben. An das erfindungsgemäße kryochirurgische Gerät 10 können starre Kryosonden 1 und flexible Kryosonden 2 angeschlossen werden. Die Steuerung 13 regelt die Druckeinstellvorrichtung 14 und die Schaltvorrichtung 15 entsprechend.

### Erstes Ausführungsbeispiel

Die Fig. 2 zeigt eine erste beispielhafte Ausgestaltung der Druckeinstellvorrichtung 14 und der Schaltvorrichtung 15. Diese umfasst genau ein 3/2-Proportionalventil 30 und ein 3/2-Schaltventil 50. Ein erster Proportionalventilanschluss 31 des Proportionalventils 30 ist mit der Fluidquelle 11, ein zweiter Proportionalventilanschluss 32 mit der Entlüftungseinrichtung 40 verbunden. Die Entlüftungseinrichtung bzw. Fluidabführeinrichtung 40 umfasst einen Schalldämpfer 41 und einen Flowsensor 42. Sie dient zum Ableiten des Fluids. Der dritte Proportionalventilanschluss 33 des Proportionalventils 30 ist an den ersten Kryosondenanschluss 21 angeschlossen. Der erste Kryosondenanschluss 21 hat eine Doppelfunktion. In einem ersten Modus ist an diesen der Rücklauf einer starren Kryosonde 1 angeschlossen, in einem zweiten Modus der Zulauf einer flexiblen Kryosonde 2.

Weiterhin steht ein erster Schaltventilanschluss 51 des 3/2-Schaltventils 50 mit dem ersten Proportionalventilanschluss 31 in Fluidverbindung. Der zweite Schaltventilanschluss 52 führt über eine Drossel 17 zu der Entlüftungseinrichtung 40 und dem zweiten Proportionalventilanschluss 32. An diese Leitung ist ebenfalls ein dritter Kryosondenanschluss 23 für den Rücklauf der flexiblen Kryosonde 2 angekoppelt. Der dritte Schaltventilanschluss 53 steht mit einem zweiten Kryosondenanschluss 22 für den Zulauf der starren Kryosonde 1 in Verbindung.

Die Steuerung 13 steuert die Drucklufteinstellvorrichtung 14 und die Schaltvorrichtung 15 gemäß der Fig. 2 derart, dass in einem ersten Modus die starre Kryosonde 1, in einem zweiten Modus eine flexible Kryosonde 2 anschließbar ist. Um die Kühlleistung der Kryosonden 1, 2 entsprechend einer Benutzereingabe einstellen zu können, weist das erfindungsgemäße kryochirurgische Gerät 10 Sensoren 19, 19', 42 auf, die den Druck am ersten Proportionalventilanschluss 31 und am dritten Proportionalventilanschluss 33 bestimmen. Die Sensoren 19, 19', 42 geben entsprechende Sensorsignale an die Steuerung 13 ab, die mit entsprechenden Regelsignalen das Proportionalventil 30 und das Schaltventil 50 einstellt. Das Proportionalventil 30 und das Schaltventil 50 weisen hierfür entsprechende Aktuatoren auf.

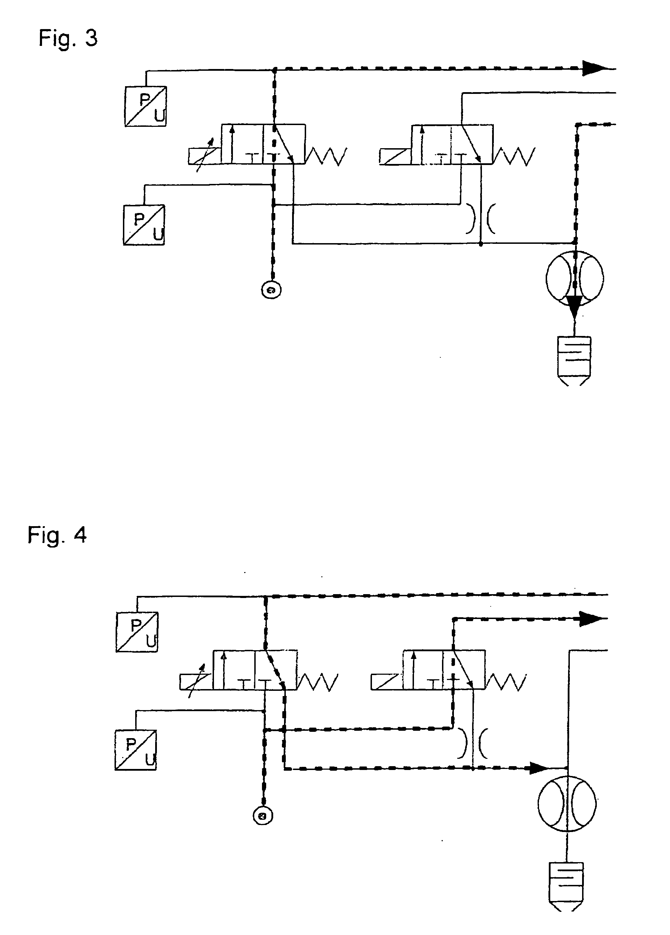

Fig. 3 zeigt die Fluidströme im zweiten Modus bzw. im Vorderdruckregelungsmodus bei angeschlossener flexibler Kryosonde 2 und bei maximaler Kühlleistung. Die flexible Kryosonde 2 ist an den ersten Kryosondenanschluss 21 und den dritten Kryosondenanschluss 23 angeschlossen. Das Proportionalventil 30 wird durch die Steuerung 13 derart aktiviert, dass das Fluid aus der Fluidquelle 11 ungedrosselt über den ersten Proportionalventilanschluss 31 zu dem Proportionalventil 32 und von dort in den Zulauf der Kryosonde 2 fließt. Der Rücklauf der flexiblen Kryosonde 2 mündet unmittelbar in die Entlüftungseinrichtung 40. Das Schaltventil 50 ist deaktiviert, d.h. es liegt keine Fluidverbindung zwischen erstem Schaltventilanschluss 51 und drittem Schaltventilanschluss 53 vor. Um einen ungewollten schnellen Abfluss des Fluids durch den zweiten Kryosondenanschluss 22 zu vermeiden, ist dort die Drossel 17 vorgesehen. Durch ein Einstellen des Proportionalventils 30 kann die Kühlleistung der flexiblen Kryosonde 2 gedrosselt werden. Sobald das Proportionalventil 30 deaktiviert ist (keine Fluidverbindung zwischen erstem und drittem Proportionalventilanschluss 30, 33), wird die flexible Kryosonde 2 nicht mehr gekühlt.

Im ersten Modus bzw. im Hinterdruckregelungsmodus (vgl. Fig. 4) bei angeschlossener starrer Kryosonde und maximaler Kühlleistung ist das Proportionalventil 30 aktiviert. Somit liegt eine Fluidverbindung zwischen zweitem Kryosondenanschluss 22 und Fluidquelle 11 vor. Das Fluid kann ungedrosselt in den Zulauf der starren Kryosonde 1 fließen. Durch ein Einstellen des Proportionalventils 30 lässt sich der Rücklauf der starren Kryosonde 1 regeln. Wie in Fig. 4 gezeigt, wird die maximale Kühlleistung durch eine Deaktivierung des Proportionalventils 30 erreicht. In dieser Ventilstellung fließt das Fluid ungedrosselt aus dem Rücklauf der starren Kryosonde 1 in die Entlüftungseinrichtung 40. Der Abfluss wird mittels des Flowsensors 42 gemessen. Durch das Einstellen des Proportionalventils 30 lässt sich die Kühlleistung, wie auch der Fluidfluss drosseln. In einer Position des Proportionalventils 30 (starke Drosselung) findet zwar noch eine Zirkulation des Fluids statt, jedoch liegt in der Expansionskammer nur noch ein unwesentlicher Druckunterschied vor, so dass hier keine Kühlleistung mehr erbracht wird. Soweit die Kryosonde 1 eine geringere Temperatur aufweist als das Fluid, wird durch dieses Kälte aufgenommen und abtransportiert. Die Fluidzirkulation führt also zu einem Abtauen der starren Kryosonde 1.

### Zweites Ausführungsbeispiel

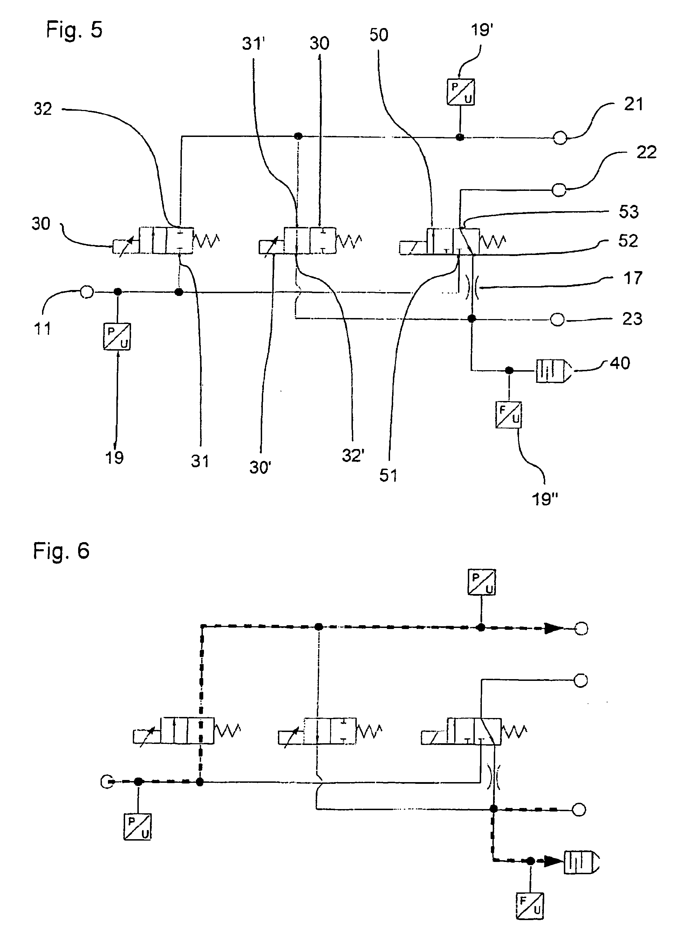

Fig. 5 zeigt eine zweite Fluidschaltung, die eine ähnliche Funktion hat wie die beschriebene erste Fluidschaltung. Das 3/2-Proportionalventil 30 und das 3/2-Schaltventil 50 sind hier jedoch durch zwei 2/2-Proportionalventile 30, 30' bzw. ein 2/2-Schaltventil 50 ersetzt. Das erste 2/2-Proportionalventil 30 hat einen ersten Proportionalventilanschluss 31 und einen zweiten Proportionalventilanschluss 32. Der erste Proportionalventilanschluss 31 ist mit der Fluidquelle 11 und der zweite Proportionalventilanschluss 32 mit dem ersten Kryosondenanschluss 21 verbunden. Das zweite Proportionalventil 30' hängt mit seinem ersten Proportionalventilanschluss 31' ebenfalls an dem ersten Kryosondenanschluss 21, während der zweite Proportionalventilanschluss 32' in fluider Verbindung mit der Entlüftungseinrichtung 40 steht. Ebenfalls an die Entlüftungseinrichtung 40 angeschlossen ist der dritte Kryosondenanschluss 23 und der zweite Schaltventilanschluss 52. Der erste Schaltventilanschluss 51 ist mit der Fluidquelle 11 und der dritte Schaltventilanschluss 53 mit dem zweiten Kryosondenanschluss 22 verbunden. Sensoren 19, 19', 19" bestimmten Messwerte an der Fluidquelle 11 bzw. dem ersten Kryosondenanschluss 21 bzw. der Entlüftungseinrichtung 40.

Im zweiten Modus (vgl. Fig. 6) ist für eine maximale Kühlleistung der flexiblen oder zweiten Kryosonde 2 das erste Proportionalventil 30 aktiviert (offen), das zweite Proportionalventil 30' deaktiviert (geschlossen) und das erste Schaltventil 50 deaktiviert (Fluidverbindung zwischen zweitem und drittem Schaltventilanschluss 52, 53). Das Fluid fließt aus der Fluidquelle 11 über das erste Proportionalventil 30 zum ersten Kryosondenanschluss 21. Der Rücklauf der flexiblen Kryosonde 2 mündet in den dritten Kryosondenanschluss 23, der unmittelbar mit der Entlüftungseinrichtung 40 verbunden ist. Eine Regulierung der Leistung der flexiblen Kryosonde 2 kann durch ein stufenweises Deaktivieren des ersten Proportionalventils 30 erzielt werden, das den Fluidzufluss je nach Position drosselt.

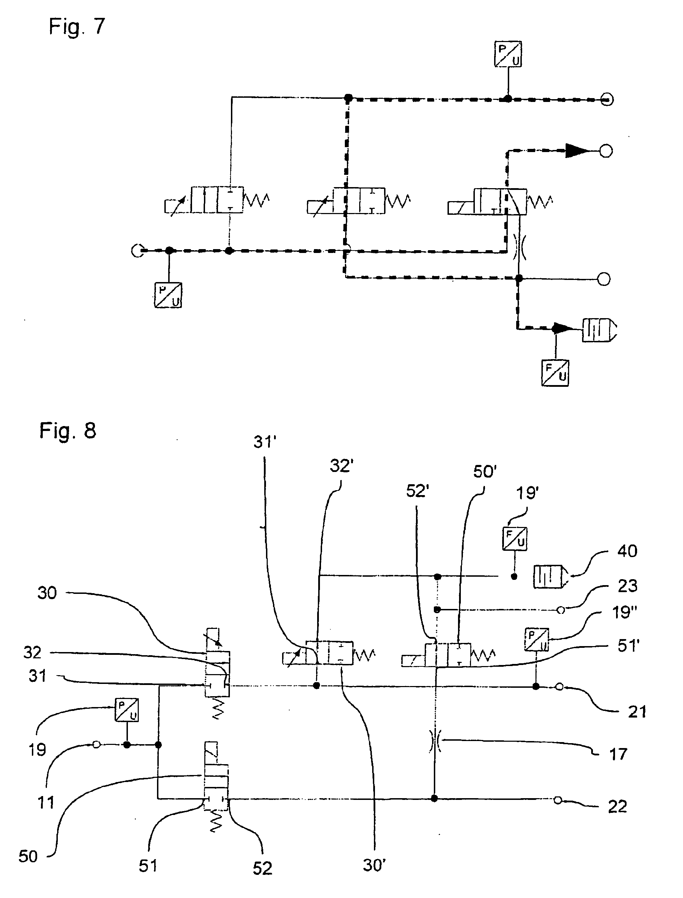

Die Maximalleistung im ersten Modus wird, wie in Fig. 7 gezeigt, dadurch erzielt, dass das erste Proportionalventil 30 deaktiviert, das zweite Proportionalventil 30' aktiviert und das Schaltventil 50 aktiviert (Fluidverbindung zwischen erstem und drittem Schaltventilanschluss 51, 53) ist. Das Fluid strömt also aus der Fluidquelle 11 ungedrosselt über das Schaltventil 50 in den zweiten Kryosondenanschluss 22 und wird von dem ersten Kryosondenanschluss 21 über das zweite Proportionalventil 30' zur Entlüftungseinrichtung 40 abgeführt. Der Druck an dem ersten Kryosondenanschluss 21 und somit die Kühlleistung der ersten oder starren Kryosonde 1 lässt sich durch das Proportionalventil 30' einstellen. Mit der Fluidschaltung gemäß Fig. 5 ist es ebenfalls möglich, den Fluidfluss im ersten Modus derart umzukehren, dass Fluid über den Rücklauf bzw. Kryosondenanschluss 21 in die starre Kryosonde 1 eingeleitet und über den Zulauf bzw. Kryosondenanschluss 22 abgeleitet wird. Die starre Kryosonde 1 kann so abgetaut werden.

### Drittes Ausführungsbeispiel

Fig. 8 zeigt eine funktionsgleiche Fluidschaltung mit zwei 2/2-Schaltventilen 50, 50' und zwei 2/2-Proportionalventilen 30, 30'. Das erste Proportionalventil 30 und das erste Schaltventil 50 sind parallel zueinander angeordnet, wobei der erste Proportionalventilanschluss 31 und der erste Schaltventilanschluss 51 in fluider Verbindung mit der Fluidquelle 11 stehen. Der zweite Proportionalventilanschluss 32 des ersten Proportionalventils 30 steht mit dem ersten Proportionalventilanschluss 31' des zweiten Proportionalventils 30' und dem ersten Kryosondenanschluss 21 in Verbindung. Der zweite Schaltventilanschluss 52 des ersten Schaltventils 50 ist mit dem zweiten Kryosondenanschluss 22 und, über eine Drossel 17, mit dem ersten Schaltventilanschluss 51' des zweiten Schaltventils 50' verbunden. Der zweite Proportionalventilanschluss 32' des zweiten Proportionalventils 30', der zweite Schaltventilanschluss 52' des zweiten Schaltventils 50' und der dritte Kryosondenanschluss 23 haben eine fluide Verbindung zu der Entlüftungseinrichtung 40. Sensoren 19 bis 19" sind entsprechend vorgesehen.

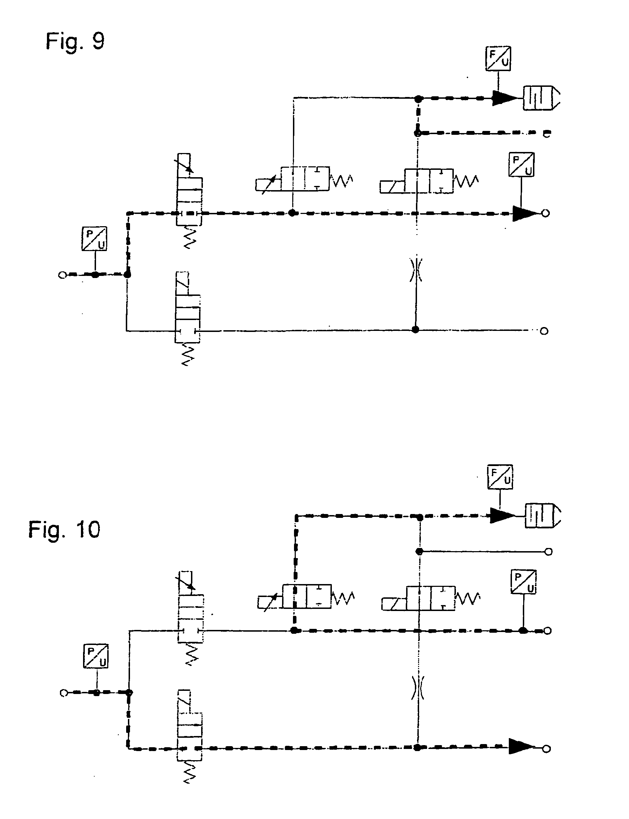

Im zweiten Modus sind, wie in Fig. 9 gezeigt, bei voller Leistung das erste Proportionalventil 31 aktiviert (völlig geöffnet) und das zweite Proportionalventil 30' deaktiviert (geschlossen). Das erste und zweite Schaltventil 50, 50' sind in diesem Modus deaktiviert. Das Fluid fließt aus der Fluidquelle 11 über das erste Proportionalventil 30 zum ersten Kryosondenanschluss 21 und wird von dem dritten Kryosondenanschluss 23 zur Entlüftungseinrichtung 40 abgeführt. Im zweiten Modus lässt sich der Fluiddruck, der am ersten Kryosondenanschluss 21 anliegt, und somit die Kühlleistung durch ein Regeln des ersten Proportionalventils 30 einstellen.

Im ersten Modus bei maximaler Leistung ist das erste Proportionalventil 30 deaktiviert, das zweite Proportionalventil 30' aktiviert, das erste Schaltventil 50 aktiviert und das zweite Schaltventil 50' deaktiviert (vgl. Fig. 10). Das Fluid fließt ungedrosselt aus der Fluidquelle 11 zum zweiten Kryosondenanschluss 22 und aus dem ersten Kryosondenanschluss 21 über das zweite Proportionalventil 30' zu der Entlüftungseinrichtung 40. Durch ein Drosseln des abfließenden Fluidflusses mittels des zweiten Proportionalventils 30' lässt sich die Leistung der ersten Kryosonde 1 einstellen.

### Viertes Ausführungsbeispiel

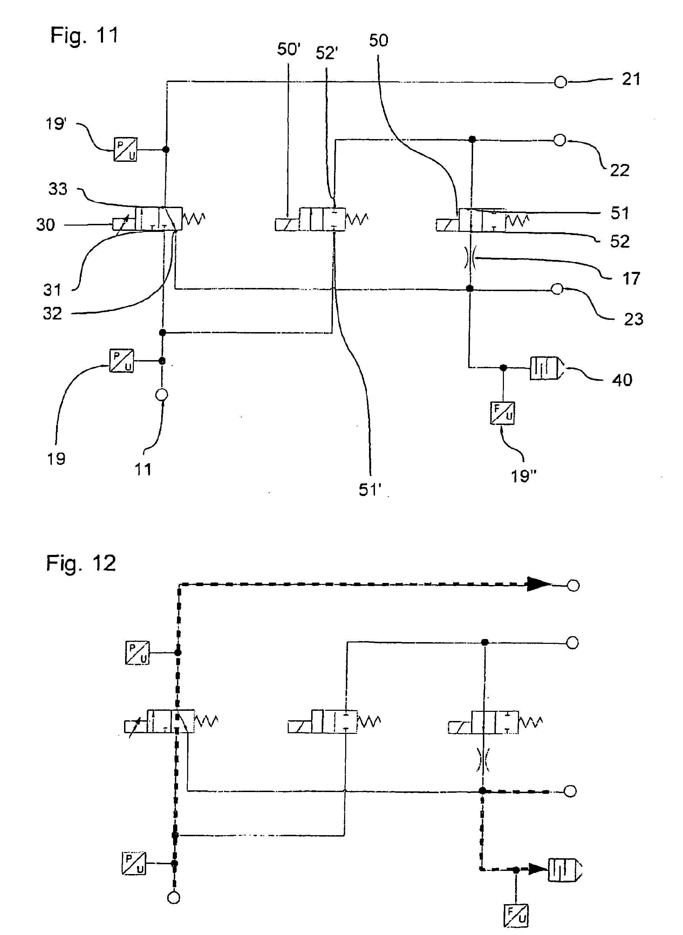

Das vierte Ausführungsbeispiel gemäß Fig. 11 weist ein 3/2-Proportionalventil 30 und zwei 2/2-Schaltventile 50, 50' auf. Der erste Proportionalventilanschluss 31 ist mit der Fluidquelle 11, der zweite Proportionalventilanschluss 32 mit der Entlüftungseinrichtung 40 und der dritte Proportionalventilanschluss 33 des Proportionalventils 30 mit dem ersten Kryosondenanschluss 21 verbunden. Weitere Verbindungen bestehen zwischen dem zweiten Kryosondenanschluss 22 und dem ersten Schaltventilanschluss 51 des ersten Schaltventils 50 sowie dem zweiten Schaltventilanschluss 52' des zweiten Schaltventils 50'. Der zweite Schaltventilanschluss 52 des ersten Schaltventils 50 ist mit der Entlüftungseinrichtung 40 und der erste Schaltventilanschluss 51 des zweiten Schaltventils 50' mit der Fluidquelle 11 verbunden. Es besteht eine fluide Verbindung zwischen dem dritten Kryosondenanschluss 23 und der Entlüftungseinrichtung 40. Sensoren 19, 19', 19" sind an der Fluidquelle 11, dem ersten Kryosondenanschluss 21 und der Entlüftungseinrichtung 40 vorgesehen. Eine Drossel 17 ist dem zweiten Schaltventilanschluss 52 des ersten Schaltventils 50 nachgeschaltet.

Im zweiten Modus sind bei maximaler Leistung das erste Proportionalventil 30 aktiviert (ungedrosselte Verbindung zwischen erstem und drittem Proportionalventilanschluss 31, 33), die beiden Schaltventile 50, 50' deaktiviert (vgl. Fig. 12). Das Fluid fließt ungedrosselt aus der Fluidquelle 11 über das Proportionalventil 30 zu dem ersten Kryosondenanschluss 21 und von dem zweiten Kryosondenanschluss 22 in die Entlüftungseinrichtung 40. Eine Regulierung des Fluidflusses kann über eine Drosselung im Proportionalventil 30 erzielt werden.

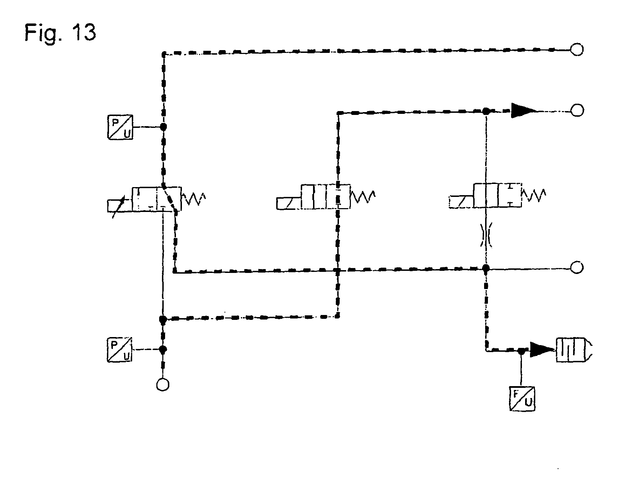

Im ersten Modus (vgl. Fig. 13) bei maximaler Leistung sind das Proportionalventil 30 deaktiviert (ungedrosselte Verbindung zwischen dem ersten und dritten Proportionalventilanschluss 32, 33), das erste Schaltventil 50 deaktiviert und das zweite Schaltventil 50' aktiviert. Das Fluid fließt aus der Entlüftungseinrichtung 40 ungedrosselt in den zweiten Kryosondenanschluss 22 und aus dem ersten Kryosondenanschluss 21 über das Proportionalventil 30 in die Entlüftungseinrichtung 40. Der Rücklauf aus der ersten Kryosonde 1 kann durch das Einstellen des Proportionalventils 30 geregelt werden. Das Proportionalventil 30 dient dann als Drossel und erhöht den Druck in der Rücklaufleitung. Die Leistung der starren Kryosonde 1 nimmt ab.

Die vorab geschilderten Ausführungsbeispiele dienen lediglich zur Illustration unterschiedlicher Schaltungsanordnungen, die den beanspruchten Effekt erzielen. Für eine marktfähige Implementierung kann es notwendig sein, weitere Drosseln 17 oder Rückschlagventile vorzusehen. Des Weiteren ist es möglich, die zweiten und dritten Kryosondenanschlüsse 22, 23 mittels eines weiteren 3/2-Schaltventils zu einem Anschluss zusammenzufassen.

Dem Fachmann sollten zahlreiche weitere Ausgestaltungsformen der Schaltungen geläufig sein, die den selben Effekt erzielen.

### Bezugszeichenliste

- 1: starre Kryosonde
- 2: flexible Kryosonde
- 10: kryochirurgisches Gerät
- 11: Fluidquelle
- 13: Steuerung
- 14: Drucklufteinstellvorrichtung
- 15: Schaltvorrichtung
- 17: Drossel
- 19, 19', 19": Sensor
- 20: Kryosondenanschlusseinrichtung
- 21: erster Kryosondenanschluss
- 22: zweiter Kryosondenanschluss
- 23: dritter Kryosondenanschluss
- 30, 30': Proportionalventil
- 31, 31': erster Proportionalventilanschluss
- 32, 32': zweiter Proportionalventilanschluss
- 33: dritter Proportionalventilanschluss
- 40: Entlüftungseinrichtung bzw. Fluidabführeinrichtung
- 41: Schalldämpfer
- 42: Flowsensor
- 50, 50': Schaltventil
- 51, 51': erster Schaltventilanschluss
- 52, 52': zweiter Schaltventilanschluss
- 53: dritter Schaltventilanschluss

## Patentansprüche

1. Kryochirurgisches Gerät zum Betreiben einer ersten Kryosonde (1) mit einem druckfesten Rücklauf und einer zweiten Kryosonde (2) mit einem drucklosen Rücklauf,
wobei das Gerät einen ersten Modus (Hinterdruckregelungsmodus) zum Betreiben der ersten Kryosonde (1) und einen zweiten Modus (Vorderdruckregelungsmodus) zum Betreiben der zweiten Kryosonde (2) hat,
wobei das Gerät umfasst:
- eine Steuerung (13);
- eine Fluidquelle (11) zum Bereitstellen eines Fluids, das zur Kühlung der Kryosonden (1, 2) mittels des Joule-Thomson-Effekts und/oder durch Verdampfen eines verflüssigten Gases in diese einleitbar ist;
- mindestens zwei Kryosondenanschlüsse (21-23), wobei an einen der Kryosondenanschlüsse (21-23) im ersten Modus der Rücklauf der ersten Kryosonde (1) und im zweiten Modus ein Zulauf der zweiten Kryosonde (2) angeschlossen ist;
- eine Druckeinstellvorrichtung (14) mit mindestens einem Druckregelventil (30, 30'), die derart mit den Kryosonden (1, 2) verbunden ist, dass durch diese im ersten und zweiten Modus das Druckverhältnis des Zulaufs zum Rücklauf der Kryosonden (1, 2) und/oder der Druck im Zulauf regelbar ist, wobei die Steuerung (13) durch ein Einstellen des Druckregelventils (30, 30') die Kühlleistung der Kryosonden (1, 2) steuert.

2. Kryochirurgisches Gerät nach Anspruch 1,
**dadurch gekennzeichnet, dass**
die Druckeinstellvorrichtung (14) im Hinterdruckregelungsmodus derart mit der ersten Kryosonde (1) verbunden ist, dass durch das Einstellen des Druckregelventils (30, 30') der Druck in dem Zulauf und in dem Rücklauf der ersten Kryosonde (1) regelbar ist.

3. Kryochirurgisches Gerät nach einem der vorhergehenden Ansprüche,
**dadurch gekennzeichnet, dass**
die Druckeinstellvorrichtung (14) im Vorderdruckregelungsmodus derart mit der zweiten Kryosonde (2) verbunden ist, dass bei im Wesentlichen offenem oder drucklosem Rücklauf der zweiten Kryosonde (2) der Druck in dem Zulauf der zweiten Kryosonde (2) regelbar ist.

4. Kryochirurgisches Gerät nach einem der vorhergehenden Ansprüche,
**dadurch gekennzeichnet, dass**
die Druckeinstellvorrichtung (14) ein 3/2-Proportionalventil (30) umfasst, das mit einem ersten Proportionalventilanschluss (31) an die Fluidquelle (11), mit einem zweiten Proportionalventilanschluss (32) an eine Fluidabführeinrichtung (40) und mit einem dritten Proportionatventilanschluss (33) an mindestens einen der Kryosondenanschlüsse (21-23) angeschlossen ist.

5. Kryochirurgisches Gerät nach einem der vorhergehenden Ansprüche,
**gekennzeichnet durch**
eine Schaltvorrichtung (15) mit mindestens einem Schaltventil (50, 50'), die für einen Wechsel zwischen dem ersten und den zweiten Modus mit der Druckeinstelleinrichtung (14) und mindestens einem Kryosondenanschluss (21-23) verbunden ist.

6. Kryochirurgisches Gerät nach Anspruch 5,
**dadurch gekennzeichnet, dass**
die Steuerung (13) Aktuatoren umfaßt und den ersten oder den zweiten Modus einstellt.

7. Kryochirurgisches Gerät nach Anspruch 5 oder 6,
**dadurch gekennzeichnet, dass**
die Schaltvorrichtung (15) ein 3/2-Schaltventil (50) umfasst, das mit einem ersten Schaltventilanschluss (51) an die Druckeinstellvorrichtung (14), mit einem zweiten Schaltventilanschluss (52) an eine Entlüftungseinrichtung (40) und mit dem dritten Schaltventilanschluss (53) an mindestens einen der Kryosondenanschlüsse (21-23) angeschlossen ist.

8. Kryochirurgischcs Gerät nach einem der vorhergehenden Ansprüche,
**gekennzeichnet durch**
mindestens einen Drucksensor (19, 19', 19") zum Bestimmen eines Eingangsdrucks und/oder eines Ausgangsdrucks der Druckeinstellvorrichtung (14), wobei die Drucksensoren (19, 19', 19") Drucksensorsignale bereitstellen, die die Steuerung zur Regelung des Druckverhältnisses zwischen Zulauf und Rücklauf oder des Drucks im Zulauf verwenden.

9. Kryochirurgisches Gerät nach einem der vorhergehenden Ansprüche,
**dadurch gekennzeichnet, dass**
das Fluid ein Gasgemisch aus Kohlendioxid (CO₂) und/oder Stickoxydul (N₂O) ist.

10. Kryochirurgisches Gerät nach einem der vorhergehenden Ansprüche,
**gekennzeichnet durch**
eine Detektionseinrichtung, die feststellt, ob die erste Kryosonde (1) oder die zweite Kryosonde (2) an die Kryosondenanschlüsse (21-23) angeschlossen ist.

11. Kryochirurgisches Gerät nach Anspruch 10,
**dadurch gekennzeichnet, dass**
die Detektionseinrichtung eine Gerätekennzeichnung der Kryosonden (1, 2) ausliest, wobei die Steuerung (13) mit der Detektionseinrichtung verbunden ist, um Gerätekennzeichensignale von der Detektionseinriclitung zu empfangen und dementsprechend den ersten oder den zweiten Modus einzustellen.

12. Kryochirurgisches Gerät nach einem der vorhergehenden Ansprüche,
**gekennzeichnet durch**
einen Flowsensor (42) zur Bestimmung der Kühlleistung der Kryosonde (1, 2).

13. Kryochirurgisches Gerät nach einem der vorhergehenden Ansprüche,
**gekennzeichnet durch**
eine Sicherungseinheit zur Detektion einer pneumatischen Verbindung zwischen mindestens einem Kryosondenanschluss (21 - 23) und der Kryosonde (1, 2), wobei die Sicherungseinrichtung eine Fluidzufuhr zu dem mindestens einen Kryosondenanschluss (21 - 23) unterbricht, wenn die pneumatische Verbindung unterbrochen ist.

14. Verfahren zum Betreiben einer Kryosonden (1, 2) mit einem kryochirurgischen Gerät,
wobei das Gerät ein Fluid zum Kühlen der Kryosonde (1, 2) bereitstellt, umfassend die Schritte:
a) Bestimmen, ob eine erste Kryosonde (1) mit einem druckfesten Rücklauf oder eine zweite Kryosonde (2) mit einem drucklosen Rücklauf angeschlossen ist;
b) Einstellen eines ersten Modus (Hinterdruckregelungsmodus), wenn die erste Kryosonde (1) angeschlossen ist, und Einstellen eines zweiten Modus (Vorderdruckregelungsmodus), wenn die zweite Kryosonde (2) angeschlossen ist;
c) Steuern einer Druckeinstellvorrichtung mit einem Druckregelventil (30) zum Regeln eines Druckverhältnisses zwischen Zulauf und Rücklauf der Kryosonden (1, 2) und/oder des Drucks im Zulauf;
wobei der Schritt b) ein derartiges Einstellen einer Schaltvorrichtung (15) umfasst, dass im ersten Modus zumindest der Rücklauf der ersten Kryosonde (1) mit einem Druckregelventilanschluss (31-33) des Druckregelventils (30) verbunden ist und im zweiten Modus zumindest der Zulauf der zweiten Kryosonde (2) mit einem Druckregelventilanschluss (31-33) des Druckregelventils (30) verbunden ist.

15. Verfahren nach Anspruch 14,
**dadurch gekennzeichnet, dass**
im ersten Modus zum Einstellen der Kühlleistung der ersten Kryosonde (1) bei konstantem Druck in dem Zulauf der Druck in dem Rücklauf geregelt wird.

16. Verfahren nach Anspruch 14 oder 15,
**dadurch gekennzeichnet, dass**
im zweiten Modus zum Einstellen der Kühlleistung der zweiten Kryosonde (2) der Druck in dem Zulauf der zweiten Kryosonde (2) geregelt wird.

## Claims

1. Cryosurgical appliance for operating a first cryoprobe (1) with a pressure-resistant return line and a second cryoprobe (2) with an unpressurized return line, wherein the appliance has a first mode (back pressure regulating mode) for operating the first cryoprobe (1) and a second mode (front pressure regulating mode) for operating the second cryoprobe (2), the appliance comprising:
- a controller (13);
- a fluid source (11) for providing a fluid, which can be introduced into the cryoprobes (1, 2) for cooling them by means of the Joule-Thomson effect and/or by evaporation of a liquefied gas;
- at least two cryoprobe connections (21-23), wherein, in the first mode, the return line of the first cryoprobe (1) is connected to one of the cryoprobe connections (21-23) and, in the second mode, a supply line of the second cryoprobe (2) is connected thereto;
- a pressure-setting device (14) with at least one pressure-regulating valve (30, 30'), which pressure-setting device (14) is connected to the cryoprobes (1, 2) in such a way as to regulate, in the first and second modes, the pressure ratio of the supply line to the return line of the cryoprobes (1, 2) and/or the pressure in the supply line, wherein the controller (13) controls the cooling power of the cryoprobes (1, 2) by setting the pressure-regulating valve (30, 30').

2. Cryosurgical appliance according to Claim 1, **characterized in that** the pressure-setting device (14), in the back pressure regulating mode, is connected to the first cryoprobe (1) in such a way that the pressure in the supply line and in the return line of the first cryoprobe (1) can be regulated by setting the pressure-regulating valve (30, 30').

3. Cryosurgical appliance according to one of the preceding claims, **characterized in that** the pressure-setting device (14), in the front pressure regulating mode, is connected to the second cryoprobe (2) in such a way that, with the return line of the second cryoprobe (2) substantially open or unpressurized, the pressure in the supply line of the second cryoprobe (2) can be regulated.

4. Cryosurgical appliance according to one of the preceding claims, **characterized in that** the pressure-setting device (14) comprises a 3/2 proportional valve (30), which is connected by a first proportional valve connection (31) to the fluid source (11), by a second proportional valve connection (32) to a fluid removal device (40), and by a third proportional valve connection (33) to at least one of the cryoprobe connections (21-23).

5. Cryosurgical appliance according to one of the preceding claims, **characterized by** a switching device (15) with at least one switching valve (50, 50'), which switching device (15) is connected to the pressure-setting device (14) and to at least one cryoprobe connection (21-23) for changing between the first and the second mode.

6. Cryosurgical appliance according to Claim 5, **characterized in that** the controller (13) comprises actuators and sets the first or the second mode.

7. Cryosurgical appliance according to Claim 5 or 6, **characterized in that** the switching device (15) comprises a 3/2 switching valve (50), which is connected by a first switching valve connection (51) to the pressure-setting device (14), by a second switching valve connection (52) to a ventilation device (40), and by the third switching valve connection (53) to at least one of the cryoprobe connections (21-23).

8. Cryosurgical appliance according to one of the preceding claims, **characterized by** at least one pressure sensor (19, 19', 19") for determining an input pressure and/or an output pressure of the pressure-setting device (14), the pressure sensors (19, 19', 19") providing pressure sensor signals, which are used by the controller for regulating the pressure ratio between supply line and return line or for regulating the pressure in the supply line.

9. Cryosurgical appliance according to one of the preceding claims, **characterized in that** the fluid is a gas mixture of carbon dioxide (CO₂) and/or nitrous oxide (N₂O).

10. Cryosurgical appliance according to one of the preceding claims, **characterized by** a detection device which ascertains whether the first cryoprobe (1) or the second cryoprobe (2) is connected to the cryoprobe connections (21-23).

11. Cryosurgical appliance according to Claim 10, **characterized in that** the detection device reads out an appliance identifier of the cryoprobes (1, 2), and the controller (13) is connected to the detection device in order to receive appliance identifier signals from the detection device and accordingly set the first or the second mode.

12. Cryosurgical appliance according to one of the preceding claims, **characterized by** a flow sensor (42) for determining the cooling power of the cryoprobe (1, 2).

13. Cryosurgical appliance according to one of the preceding claims, **characterized by** a safety unit for detecting a pneumatic connection between at least one cryoprobe connection (21-23) and the cryoprobe (1, 2), the safety device interrupting a supply of fluid to the at least one cryoprobe connection (21-23) if the pneumatic connection is interrupted.

14. Method for operating a cryoprobe (1, 2) with a cryosurgical appliance, wherein the appliance provides a fluid for cooling the cryoprobe (1, 2), said method comprising the steps of:
a) determining whether a first cryoprobe (1) with a pressure-resistant return line or a second cryoprobe (2) with an unpressurized return line is connected;
b) setting a first mode (back pressure regulating mode) if the first cryoprobe (1) is connected, and setting a second mode (front pressure regulating mode) if the second cryoprobe (2) is connected;
c) controlling a pressure-setting device with a pressure-regulating valve (30) for regulating a pressure ratio between supply line and return line of the cryoprobes (1, 2) and/or regulating the pressure in the supply line;
wherein step b) includes setting a switching device (15) in such a way that, in the first mode, at least the return line of the first cryoprobe (1) is connected to a pressure-regulating valve connection (31-33) of the pressure-regulating valve (30) and, in the second mode, at least the supply line of the second cryoprobe (2) is connected to a pressure-regulating valve connection (31-33) of the pressure-regulating valve (30).

15. Method according to Claim 14, **characterized in that**, in the first mode, for setting the cooling power of the first cryoprobe (1), the pressure in the return line is regulated at constant pressure in the supply line.

16. Method according to Claim 14 or 15, **characterized in that**, in the second mode, for setting the cooling power of the second cryoprobe (2), the pressure in the supply line of the second cryoprobe (2) is regulated.

## Revendications

1. Appareil cryochirurgical pour faire fonctionner une première cryosonde (1) avec un retour résistant à la pression et une deuxième cryosonde (2) avec un retour sans pression,
dans lequel l'appareil comporte un premier mode (mode de régulation de la pression aval) pour faire fonctionner la première cryosonde (1) et un deuxième mode (mode de régulation de la pression amont) pour faire fonctionner la deuxième cryosonde (2),
dans lequel l'appareil comprend:
- une commande (13);
- une source de fluide (11) pour fournir un fluide, qui peut être introduit dans les cryosondes (1, 2) en vue de leur refroidissement par l'effet Joule-Thomson et/ou par évaporation d'un gaz liquéfié;
- au moins deux raccords de cryosonde (21-23), dans lequel le retour de la première cryosonde (1) est raccordé dans le premier mode à un des raccords de cryosonde (21-23) et une arrivée de la deuxième cryosonde (2) est raccordée dans le deuxième mode à un des raccords de cryosonde (21-23);
- un dispositif de réglage de pression (14) avec au moins une soupape de régulation de pression (30, 30'), qui est raccordé aux cryosondes (1, 2) de telle manière que le rapport de pression de l'arrivée au retour des cryosondes (1, 2) et/ou la pression dans l'arrivée soit réglable par celui-ci dans les premier et deuxième modes, dans lequel la commande (13) commande la puissance de refroidissement des cryosondes (1, 2) par un réglage de la soupape de régulation de pression (30, 30').

2. Appareil cryochirurgical selon la revendication 1, **caractérisé en ce que**, dans le mode de régulation de pression aval, le dispositif de réglage de pression (14) est relié à la première cryosonde (1), de telle façon que la pression dans l'arrivée et dans le retour de la première cryosonde (1) soit réglable par le réglage de la soupape de régulation de pression (30, 30').

3. Appareil cryochirurgical selon l'une quelconque des revendications précédentes, **caractérisé en ce que**, dans le mode de régulation de pression amont, le dispositif de réglage de pression (14) est relié à la deuxième cryosonde (2), de telle façon que la pression dans l'arrivée de la deuxième cryosonde (2) soit réglable lorsque le retour de la deuxième cryosonde (2) est essentiellement ouvert ou sans pression.

4. Appareil cryochirurgical selon l'une quelconque des revendications précédentes, **caractérisé en ce que** le dispositif de réglage de pression (14) comprend une vanne proportionnelle 3/2 (30), qui est raccordée par un premier raccord de vanne proportionnelle (31) à la source de fluide (11), par un deuxième raccord de vanne proportionnelle (32) à un dispositif d'évacuation de fluide (40) et par un troisième raccord de vanne proportionnelle (33) à au moins un des raccords de cryosonde (21-23).

5. Appareil cryochirurgical selon l'une quelconque des revendications précédentes, **caractérisé par** un dispositif de commutation (15) avec au moins une soupape de commande (50, 50') qui, pour un changement entre le premier mode et le deuxième mode, est raccordée au dispositif de réglage de pression (14) et à au moins un raccord de cryosonde (21-23).

6. Appareil cryochirurgical selon la revendication 5, **caractérisé en ce que** la commande (13) comprend des actionneurs et règle le premier ou le deuxième mode.

7. Appareil cryochirurgical selon la revendication 5 ou 6, **caractérisé en ce que** le dispositif de commutation (15) comprend une soupape de commande 3/2 (50), qui est raccordée par un premier raccord de soupape de commande (51) au dispositif de réglage de pression (14), par un deuxième raccord de soupape de commande (52) à un dispositif d'aération (40) et par le troisième raccord de soupape de commande (53) à au moins un des raccords de cryosonde (21-23).

8. Appareil cryochirurgical selon l'une quelconque des revendications précédentes, **caractérisé par** au moins un capteur de pression (19, 19', 19") destiné à déterminer une pression d'entrée et/ou une pression de sortie du dispositif de réglage de pression (14), dans lequel les capteurs de pression (19, 19', 19") produisent des signaux de capteurs de pression, que la commande utilise pour la régulation du rapport de pression entre l'arrivée et le retour ou de la pression dans l'arrivée.

9. Appareil cryochirurgical selon l'une quelconque des revendications précédentes, **caractérisé en ce que** le fluide est un mélange gazeux de dioxyde de carbone (CO₂) et/ou de protoxyde d'azote (N₂O).

10. Appareil cryochirurgical selon l'une quelconque des revendications précédentes, **caractérisé par** un dispositif de détection, qui constate si la première cryosonde (1) ou la deuxième cryosonde (2) est raccordée aux raccords de cryosonde (21-23).

11. Appareil cryochirurgical selon la revendication 10, **caractérisé en ce que** le dispositif de détection lit une identification d'appareil des cryosondes (1, 2), dans lequel la commande (13) est reliée au dispositif de détection pour recevoir des signaux d'identification d'appareil émanant du dispositif de détection et pour régler de manière correspondante le premier ou le deuxième mode.

12. Appareil cryochirurgical selon l'une quelconque des revendications précédentes, **caractérisé par** un détecteur de débit (42) destiné à déterminer la puissance de refroidissement des cryosondes (1, 2).

13. Appareil cryochirurgical selon l'une quelconque des revendications précédentes, **caractérisé par** une unité de sécurité pour la détection d'une communication pneumatique entre au moins un raccord de cryosonde (21-23) et la cryosonde (1, 2), dans lequel le dispositif de sécurité interrompt une arrivée de fluide audit au moins un raccord de cryosonde (21-23), lorsque la communication pneumatique est interrompue.

14. Procédé pour faire fonctionner une cryosonde (1, 2) avec un appareil cryochirurgical,
dans lequel l'appareil fournit un fluide pour le refroidissement de la cryosonde (1, 2),
comprenant les étapes suivantes:
a) déterminer si une première cryosonde (1) avec un retour résistant à la pression ou une deuxième cryosonde (2) avec un retour sans pression est raccordée;
b) régler un premier mode (mode de régulation de pression aval) lorsque la première cryosonde (1) est raccordée, et régler un deuxième mode (mode de régulation de pression amont), lorsque la deuxième cryosonde (2) est raccordée;
c) commander un dispositif de réglage de pression avec une soupape de régulation de pression (30) pour réguler un rapport de pression entre l'arrivée et le retour des cryosondes (1, 2) et/ou de la pression dans l'arrivée;
dans lequel l'étape b) comprend un réglage d'un dispositif de commutation (15) de telle manière que, dans le premier mode, au moins le retour de la première cryosonde (1) soit raccordé à un raccord de soupape de régulation de pression (31-33) de la soupape de régulation de pression (30) et que, dans le deuxième mode, au moins l'arrivée de la deuxième cryosonde (2) soit raccordée à un raccord de soupape de régulation de pression (31-33) de la soupape de régulation de pression (30).

15. Procédé selon la revendication 14, **caractérisé en ce que** dans le premier mode, on régule la pression dans le retour pour le réglage de la puissance de refroidissement de la première cryosonde (1) avec une pression constante dans l'arrivée.

16. Procédé selon la revendication 14 ou 15, **caractérisé en ce que** dans le deuxième mode, on régule la pression dans l'arrivée de la deuxième cryosonde (2) pour le réglage de la puissance de refroidissement de la deuxième cryosonde (2).
